**Europäisches Patentamt**

**European Patent Office**

**Office européen des brevets

(11) Veröffentlichungsnummer: **0 322 667 B1**

(19)

(12) **EUROPÄISCHE PATENTSCHRIFT**

(45) Veröffentlichungstag der Patentschrift: **16.09.92**

(21) Anmeldenummer: **88120986.0**

(22) Anmeldetag: **15.12.88**

(51) Int. Cl.⁵: **E02D 29/02**, E02D 17/20, A01G 9/02, E01F 8/00

(54) Verfahren zur Herstellung einer Mauer, Bauelement und Verbindungsplatte zur Ausführung des Verfahrens und Mauer hergestellt nach dem Verfahren.

(30) Priorität: **31.12.87 CH 5120/87**

(43) Veröffentlichungstag der Anmeldung:
**05.07.89 Patentblatt 89/27**

(45) Bekanntmachung des Hinweises auf die
Patenterteilung:
**16.09.92 Patentblatt 92/38**

(84) Benannte Vertragsstaaten:
**AT CH DE FR IT LI**

(56) Entgegenhaltungen:
EP-A- 0 034 565    EP-A- 0 065 199
EP-A- 0 215 991    EP-A- 0 215 994
DE-A- 3 236 739    DE-A- 3 401 629
DE-A- 3 406 136

(73) Patentinhaber: **Kalbermatten, Otto**
**Bergjiweg 5**
**CH-3930 Visp(CH)**

(72) Erfinder: **Kalbermatten, Otto**
**Bergjiweg 5**
**CH-3930 Visp(CH)**

(74) Vertreter: **Keller, René, Dr. et al**
**Patentanwälte Dr. René Keller & Partner**
**Postfach 12**
**CH-3000 Bern 7(CH)**

EP 0 322 667 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung einer Mauer gemäß dem Oberbegriff des Patentanspruchs 1, ein Bausatz zur Ausführung des Verfahrens gemäß dem Oberbegriff der Patentanspruchs 8, und eine Mauer hergestellt nach dem Verfahren gemäß dem Oberbegriff des Patentanspruchs 17.

Mauern, insbesondere Hangsicherungsmauern dieser Art, wie sie aus der CH-A 612 238 bekannt sind, bestehen aus Hohlsteinen mit zwei durch eine Versteifungswand getrennten Kammern, die mit Erde gefüllt werden. Die Mauer wird nach Fertigstellung mit Pflanzen begrünt, so daß sie sich gut in das bestehende Landschaftbild eingliedert. Sie besitzt eine gewisse Standfestigkeit durch das Verzahnen der Hohlsteine, das Gewicht der Erde in den Hohlsteinen und die klammernden Kräfte der Wurzeln der Bepflanzung; große Hangschubkräfte kann sie aber nicht aufnehmen.

Aus der EP-A-0 215 994 ist eine Mauer bekannt, die aus Trogsteinen und Verbindungsplatten aufgebaut wird. Die Trogsteine weisen einen einzigen, zusammenhängenden Hohlraum auf. Zum Aufbauen der Mauer werden benachbarte Trogsteine durch eine seitenmittig angeordnete Verbindungsplatte verbunden. Der Trogstein ist am Boden durch zwei Bodenplatten verschlossen (Spalte 3, letzte Zeile). Die Fixierung der Mauer geschieht durch die Schwalbenschwanz-Verbindung zwischen Trogstein und Verbindungsplatte. Um den Hang besser stabilisieren zu können, werden die Trogsteine nur mit einer Bodenplatte verlegt, so dass die Pflanzen ihre Wurzeln aus dem Trogstein hinaus in den Hang hinein verlängern können.

Der Erfindung liegt die Aufgabe zugrunde, eine möglichst steile, bepflanzbare Mauer hoher Eigenstabilität mit hoher Austrocknungsresistenz und mit guten Belüftung- und Entwässerungsmöglichkeiten des dahinterliegenden Gebietes herzustellen.

Die erfindungsgemäße Lösung dieser Aufgabe ist in verfahrensmäßiger Hinsicht durch die im Anspruch 1, hinsichtlich des Bausatzes zur Ausführung des Verfahrens durch die in dem Anspruch 8 und hinsichtlich der nach dem Verfahren hergestellten Mauer durch die im Anspruch 17 angegebenen Merkmale gekennzeichnet.

Im folgenden wird ein Ausführungsbeispiel der Erfindung anhand der Zeichnung näher erläutert. Es zeigen:

Fig. 1    eine perspektivische Darstellung einer Hangsicherungsmauer,

Fig. 2    eine Draufsicht auf ein Bauelement mit eingelegter Winkelplatte,

Fig. 3    einen Schnitt entlang der Linie III - III in Fig. 2,

Fig. 4    eine Vorderansicht einer Verbindungsplatte in Blickrichtung IV in Fig. 1,

Fig. 5    einen Schnitt entlang der Linie V - V in Fig. 4 durch die Verbindungsplatte,

Fig. 6    eine Draufsicht auf die Verbindungsplatte

Fig. 7    einen Schnitt entlang der Linie VII - VII in Fig. 1 durch die Hangsicherungsmauer,

Fig. 8    eine Draufsicht auf eine Winkelplatte,

Fig. 9    eine Seitenansicht der Winkelplatte,

Fig. 10    einen Fig. 3 entsprechenden Schnitt durch eine Variante des Bauelements und

Fig. 11    eine Draufsicht auf übereinander gestapelte Bauelemente einer frei stehenden Mauer.

Die Hangsicherungsmauer 1 wird aus Bauelementen 2, Winkelplatten 18 und Verbindungsplatten 3 aufgebaut. Im folgenden werden zuerst die Bauelemente 2, die Winkelplatten 18 und die Verbindungsplatten 3, und dann das Verfahren zur Herstellung einer Hangsicherungsmauer beschrieben.

Das Bauelement 2 ist ein Betongußelement. Es besteht aus einer Vorder-4, einer Rückwand 5, zwei Seitenwänden 6 und einer Zwischenwand 7 zwischen der Vorder- 4 und der Rückwand 5. Die beiden Seitenwände 6 sind parallel zueinander und stehen senkrecht zur Rück- 5, und Zwischenwand 7, sowie zum mittleren Teil der Vorderwand 4. Die Vorderwand 4 hat drei Teilflächen, wobei ein vorderer mittlerer Teil, der parallel zur Rückwand 5 liegt und je zwei anschließende Flächen, die unter 45° zu den Seitenwänden abgewinkelt sind, vorhanden sind. Die Rückwand 5, die Seitenwände 6 und die Vorderwand 4 bilden zusammen ein geschlossenes Vieleck. Das Bauelement 2 ist an seiner Ober- und Unterseite vollständig offen. Die Vorderwand 4 ist aus ästhetischen Gründen mit Längsriefen 8 an ihren Außenflächen ausgestattet. Die Zwischenwand 7 bildet eine Trennwand zwischen einer Kammer 9 und einer Kammer 10. Die Kammer 9 hat die Vorderwand 4 als eine ihrer Begrenzungsflächen, und die Kammer 10 die Rückwand 5. Die Kammer 10 hat in jeder ihrer Seitenwände 6 je zwei auf einer horizontalen Mittellinie liegende, annähernd miteinander fluchtende Löcher 12. Der Durchmesser der Löcher 12 ist so groß gewählt, daß Armierungseisen 14 hindurch gesteckt werden können. Am unteren Rand der Innenseite der Kammer 9 ist ein Absatz 15 zur Aufnahme einer Winkelplatte 18 vorhanden.

Beide Seitenwände 6 haben an ihren Außenseiten gegenüber der Trennwand 7 und der Rückwand 5 je eine Nut 16, die von der oberen Kante der Seitenwand 6 bis zur unteren Kante läuft. Die Nuten 16 sind sowohl an ihrem oberen, als auch an ihrem unteren Ende offen. Die Tiefe der Nuten 16 liegt etwa zwischen einem Drittel und der Hälfte der Wandstärke der Seitenwand 6. Eckenverstärkungen 19 sind an den Stoßkanten zwischen den Seitenwänden 6 und der Trennwand 7, und zwischen den Seitenwänden 6 und der Rückwand 5 ausgebildet. Aufgrund einer äußeren Eckenverstärkung 22 an jeder der äußeren Stoßkanten zwischen der Rückwand 5 und den Seitenwänden 6 ist die Rückwand 5 um die Breite einer äußeren Eckerstärkung 22 in das Bauelement 2 nach innen versetzt. Die Eckverstärkungen 19 und 22 liegen jeweils gegenüberliegend zu den Nuten 16 in der Kammer 9 bzw. 10, bzw. an der Außenseite der Rückwand 5. Sie gewährleisten um die Nuten 16 herum eine ausreichende Stabilität des Bauelements 2.

Die Verbindungsplatte 3 ist ebenfalls ein Betongußteil. Ihre Höhe und ihre Wandsstärke ist bis auf eine Toleranz gleich der der Seitenwände 6. Ihre Länge entspricht der Summe aus der Länge der Trennwand 7 und der doppelten Fugentiefe verringert durch eine Montagetoleranz. Entlang ihres oberen und unteren Längsrandes hat die Verbindungsplatte 3 je einen integrierten Ansatz 24 und 25 senkrecht an je einer der Plattenflächen. An den beiden Plattenstirnseiten verjüngen sich die Ansätze 24 und 25 bis auf die Materialstärke der Verbindungsplatte 3, damit die Verbindungsplatte in die Nuten 16 eingeschoben werden kann. Die Breite der Ansätze 24 und 5 ist durch den Versatz der übereinander angeordneten Reihen aus Bauelementen 2 und Verbindungsplatten 3, wie unten beschrieben, gegeben und ist etwas größer als die Wandstärke der Bauelemente 2.

Die Rückwand 5, die Trennwand 7 und die Verbindungsplatten 3 haben in der Mitte ihrer Längsseite an deren Ober- und Unterkante je eine Aussparung 20 bzw. 21 zum Einlegen eines Rohrstückes 27.

Die Rückwand 5 hat am unteren Rand in den äußeren Ecken je einen Vorsprung 17, der beim Aufbau der Mauer 1 als Anschlag, im Sinne einer Montagehilfe, an der Oberkante der Rückwand 5 des unter dem Bauelement 2 versetzt liegende Bauelements 2 der unteren Reihe dient.

Die Winkelplatte 18 ist ein Betongußteil deren einer Schenkel eine Bodenplatte 26 und deren anderer Schenkel eine Stützplatte 28 bildet. Die Kontur der Bodenplatte 26 ist an ihrer der Stützplatte 28 abgewandten Seite bis auf eine Toleranz gleich der zur Maueraußenseite zugewandten Innenseite der Kammer 9. Die Höhe der Stützplatte 28 ist abhängig von der Höhe des Bauelements 2 und dem Abstand der Vorderfläche der Kammer 9 von der Trennwand 7; sie beträgt im Beispiel annähernd ein Drittel der Höhe des Bauelements 2. Die Materialstärke der Winkelplatte 18 beträgt einige Zentimeter; sie ist dick genug um das Gewicht der feuchten Erde 11 und der Bepflanzung zu tragen.

Zur Herstellung der Hangsicherungsmauer 1 werden die hohlen Bauelemente 2 entlang eines horizontalen ebenen, planieren Absatzes als Fundament am Fuß des Hanges abwechselnd mit zwei Verbindungsplatten 3 verlegt. Die Bauelemente 2 werden mit ihren Seitenwänden 6 etwa senkrecht zur Hangfläche und mit ihrer Kammer 10 zum Hang gewandt, verlegt. In die beiden seitlichen Nuten 16 wird je eine Verbindungsplatte 3 eingefügt. An diese beiden Verbindungsplatten 3 wird anschließend wieder ein Bauelement 2 mit seinen Nuten 16 angefügt. Dieses abwechselnde Aneinanderfügen von Bauelementen 2 und Verbindungsplatten 3 wird fortgesetzt bis die Breite der gewünschten Hangsicherung erreicht ist.

Durch die Löcher 12 in den Seitenwänden 6 werden Armierungseisen 14 gesteckt, deren Länge von der Breite der Hangsicherungsmauer bzw. bei gebogener Mauer von deren Radius abhängt.

In jede der oberen Aussparungen 20 werden Rohre 27 gelegt. Die Länge der Rohre 27 richtet sich danach, wie weit sie in den Hang hineingesteckt werden müssen. Ihre minimale Länge ist so groß wie die Tiefe der Kammer 10 bzw. die Tiefe der Kammer 23 plus Versatz einer horizontalen Reihe zur anderen, wie weiter unten beschrieben.

In der zweiten Reihe der aufzurichtenden Hangsicherungsmauer 1 werden die Bauelemente 2 derart über die Verbindungsplatten 3 gesetzt, daß sie mit dem Vorsprung 17 an der Oberkante der bergseitigen Verbindungsplatte 3 bergseitig anliegen. Da der obere Ansatz 24 kurz vor jeder der beiden Plattenstirnseiten verjüngt ist, liegt der Vorsprung 17 nicht am Ansatz 24 an. Die Bauelemente 2 liegen dann immer auf je einer Seitenwand 6 der beiden unter ihnen seitlich versetzt leigenden Bauelemente 2 der unteren Reihe. Der untere Ansatz 25 der Verbindungsplatten 3 der zweiten Reihe deckt den Zwischenraum zwischen den Verbindungsplatten 3 der zweiten Reihe und der Trennwand 7 bzw. der Rückwand 5 des Bauelements 2 der ersten Reihe ab und der obere Ansatz 24 de Verbindungsplatten 3 der untersten Reihe den Zwischenraum zwischen den Verbindungsplatten 3 der untersten und der Trennwand 7 bzw. der Rückwand 5 der Bauelemente 2 der zweiten Reihe. Die Winkelplatten 18 werden mit ihrer Stützplatte 28 nach unten und mit ihrer Bodenplatte 26 bündig an der Innenseite der vorderen Wandung der Kammer 9 anliegend in die Kammer 9 eingelegt. Anschließend werden in die Löcher 12 der rückseitigen Kammern 10 der Bauelemente 2 wieder Armierungseisen 14 gesteckt.

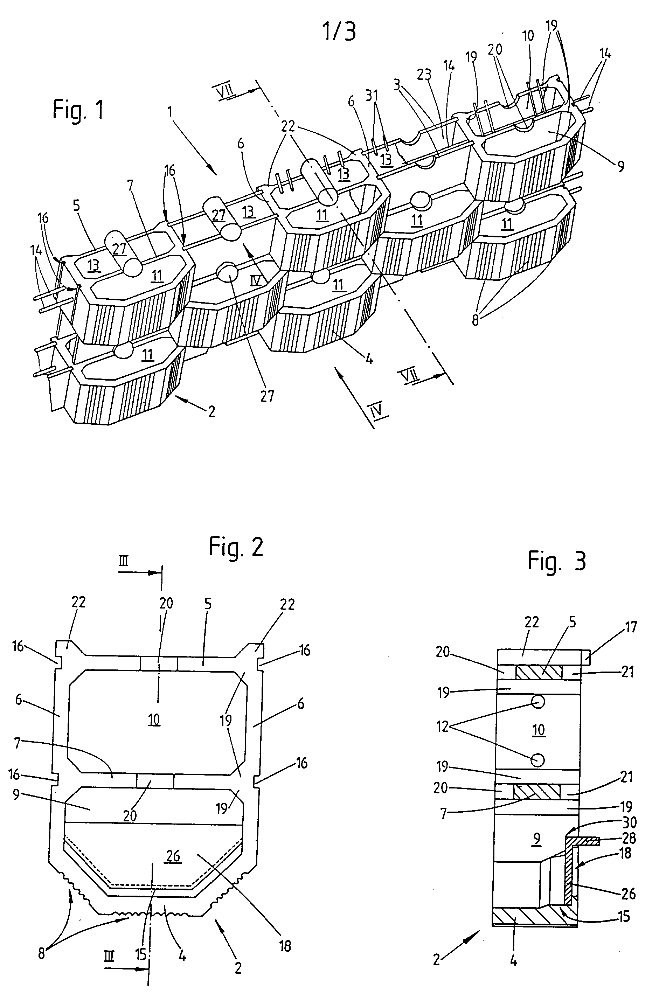

Nach der zweiten Reihe folgt eine dritte, eine vierte, usw.. Die Bauelemente 2 liegen übereinander immer auf Lücke. Etwa nach der dritten, vierten oder fünften Reihe werden in die Kammern 10 und 23 weitere Armierungeisen31 (in Figur 1 nur teilweise dargestellt) von oben nach unten gesteckt und an den horizontalen Armierungseisen 14 gegen seitliches Verrutschen mit Bindedraht gesichert. Anschließend wird in die Kammern 10 und 23 der obersten Reihe Beton 13 eingefüllt, der auch die darunterliegenden Kammern 10 und 23 füllt, und falls notwendig mit einem Vibrator verdichtet. Die Ansätze 24 und 25 an den Verbindungsplatten verhindern ein Herausquellen des Betons 13 aus den Zwischenräumen zwischen der Trennwand 7 bzw. der Rückwand 5 und den darunter bzw. darüberliegenden Verbindungsplatten 3. Analog wird mit den weiteren Reihen der Hangsicherungsmauern verfahren.

Die aufgerichtete Mauer 1 soll begrünt werden. Ein gutes Gedeihen von Pflanzen setzt u. a. ausreichende Feuchtigkeit und Belüftung in einem ausreichenden großen Erdvolumen voraus. Aus diesem Grunde wird die Mauer 1 so aufgebaut, daß übereinanderliegende Kammern 9 untereinander eine Erdverbindung besitzen und über Rohrstücke 27 mit dem hinter der Mauer 1 liegenden Hang verbunden sind.

Die Erde 11 zur Bepflanzung der Mauer 1 kann während des Aufbaus der Mauer 1 oder erst nach deren Fertigstellung in die Kammern 9 gefüllt werden, wobei die Bodenplatte 26 der in die Kammer 9 eingelegten Winkelplatte 18 die Erde 11 im vorderen Teil der Kammer 9 zurückhält. Je länger die Stützplatte 28 und je tiefer die Bodenplatte 26 in die Kammer 9 hineinragt, desto größer kann die Höhe der Bauelemente 2 sein, ohne daß Erde 11 aus der Kammer 9 über die Maueraußenseite herausfällt. Die Oberfläche der Erde 11 auf der Kammer 9 steigt schräg in Richtung Trennwand 7 zur unteren Kante der Stützplatte 28 hin an. Die Stützplatte 28 stellt einen mit Erde 11 gefüllten Kanal zwischen der Erde 11 in der Kammer 9 und der Erde 11 in bzw. auf der Kammer 9 der übernächsten unteren Reihe her, damit einerseits die Wurzeln der Pflanzen bis in die unteren Kammern 9 vordringen können und andererseits ein Feuchtigkeitsaustausch zwischen den übereinanderliegenden Kammern 9 gegeben ist.

Die Rohrstücke 27 verlaufen bei fertiggestellter Hangsicherungsmauer 1 von der Erde 11 in den Kammern 9 zum Erdreich des hinter der Mauer 1 liegenden Hangs und sind teilweise oder vollständig mit Erde gefüllt. Diese Rohrstücke 27 bewirken die Entwässerung des Hanges und verringern hierdurch den Hangdruck auf die Mauer 1, belüften das Erdreich hinter der Mauer 1 und befeuchten die Erde 11 in den Kammern 9, um eine optimale Bewachsung zu garantieren. Auch können die Wurzeln durch die Rohrstücke 27 in den Hang eindringen. Regenwasser oder Wasser aus dem Hang läuft über die Winkelplatten 18 und die Kammern 9 an der Mauer 1 nach unten. Zur Bewässerung der Erde 11 in den Kammern 9 genügt es deshalb, nur die oberste Reihe zu bewässern. Dazu kann über der oberen Reihe ein gelochtes Bewässerungsrohr verlegt werden.

Durch das Verlegen von Armierungseisen 14, 31 und Ausgießen der Kammern 10 und 23 mit Beton 13 vereinigt die Mauer 1 als Hangsicherungsmauer die hohe Stabilität einer Schwergewichtsmauer, verstärkt durch eine Armierung, mit den Vorteilen einer Mauer aus Hohlsteinen, wie u. a. Begrünbarkeit, aufgelockertes landschaftsfreundliches Erscheinungsbild und ergibt zusätzlich durch einsetzbare Rohrstüche 27 eine einfache Entwässerungs- und Belüftungsmöglichkeit des dahinterliegenden Hanges, sowie eine Befeuchtung der an der Maueraußenseite liegenden Bepflanzung.

Ist anstelle des ebenen, planierten Absatzes am Fuß des Hanges ein festes Fundament notwendig, kann wie schon oben beschrieben eine Reihe aus Bauelementen 2 und Verbindungsplatten 3, verlegt, armiert und die Kammern 9 und 10 der Bauelemente 2 sowie die Kammer 23 mit Beton ausgegossen werden. Hierauf kann dann die Mauer 1 aufgebaut werden.

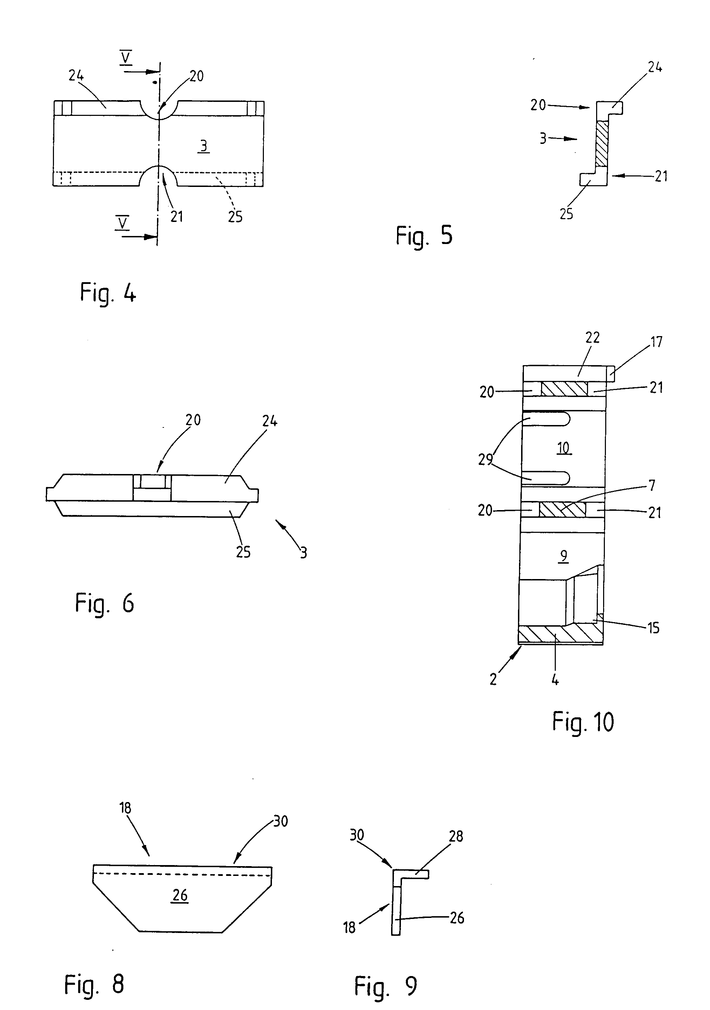

Anstelle der Löcher 12 können als Variante - wie in Fig. 10 dargestellt - in den Seitenwänden 6 Schlitze 29 vorhanden sein, in die von oben her die Armierungseisen 14 eingelegt werden können.

Anstelle der beiden Aussparungen 20, 21 in der Rückwand 5, der Trennwand 7 und den beiden Verbindungsplatten 3 in der Mitte ihrer Längsseite an deren Ober- und Unterkante zum Einlegen eines Rohrstückes 27 kann auch nur jeweils eine entsprechend größere Aussparung entweder an der Ober-oder Unterkante vorhanden sein. Bei relativ hohem Wasseranfall des Hanges kann das Rohrstück über die Vorderfläche der Mauer 1 hinausgezogen werden, damit das Wasser direkt abfließen kann. Ebenfalls kann der Spalt zwischen der Kante 30 der Winkelplatte 18 und der Trennwand 7 mit grobkörnigem Kies gegen das Auswaschen von Erde 11 geschlossen werden.

Die Rückwand 5 des Bauelements 2 kann ebenso wie die hangseitige zweite Verbindungsplatte 3 ganz oder teilweise weggelassen werden. Der einzufüllende Beton 13 wird dann von der Trennwand 7 und dem Hang, bzw. von der vorderen Verbindungsplatte 3 und dem Hang begrenzt.

Die Ansätze 24 und 25 an den Verbindungsplatten 3 zum Abdichten der Zwischenräume zwischen der Rückwand 5 und der Verbindungsplatte 3, bzw. der Trennwand 7 und der anderen Verbindungsplatte 3 in den übereinanderliegenden Reihen gegen auslaufenden Beton 13, können weggelassen werden. Um die Abdichtung dennoch zu ermöglichen, können die Nuten 16 an den Seitenwänden 6 oben und unten um die Breite des Ansatzes 24 bzw. 25 geneigt und die Höhe der Verbindungsplatten entsprechend verlängert werden.

Aufgrund der guten Standfestigkeit der Mauer 1 kann sie auch senkrecht ohne Versatz hochgezogen werden. Auch bei dieser Mauer werden die Kammern 9 mit Erde gefüllt und mit Pflanzen versehen.

Ebenfalls können zwei Wände so gegeneinander aufgestellt werden, daß die Kammern 9 jeweils nach außen zeigen und damit eine vordere und eine hintere bepflanzbare Fläche entsteht. Eine solche freistehende Mauer läßt sich z. B. als Sichtblende oder Lärmschutzwand verwenden. Dabei können Schlitze in der Rückwand 5 der Bauelemente 2 vorgesehen werden, wie sie bei der Variante in Fig. 10 für die Seitenwände 6 dargestellt sind, in die weitere Armierungseisen eingelegt werden, die die beiden Mauerhälften aus identischen Bauelementen 2 miteinander verankern.

In den Zwischenraum zwischen den Rückwänden 5 der Bauelemente 2 der Wände kann Erde als Feuchtigkeitsspeicher eingefüllt werden. Die Bauelemente können als Variante derart gestaltet sein, daß in der Rückwand zwei Aussparungen für Rohrstücke und in der Trennwand zwischen den beiden Kammern eine Aussparung für eines der beiden Rohrstücke vorhanden sind. Jeweils ein Rohrstück endet in der der Außenwand abgewandten Kammer, und das andere Rohrstück in der Kammer an der Außenwand. Das erste Rohrstück verbindet die beiden zur Außenwand abgewandten Kammern miteinander und dient als Durchführung für eine Armierung zur Verklammerung der Kammern. Das zweite Rohrstück verbindet die Kammern an der Außenwand mit der Erde zwischen den Wänden und sorgt, nachdem diese Kammern mit Erde gefüllt und bepflanzt sind, für den notwendigen Feuchtigkeitsaustausch.

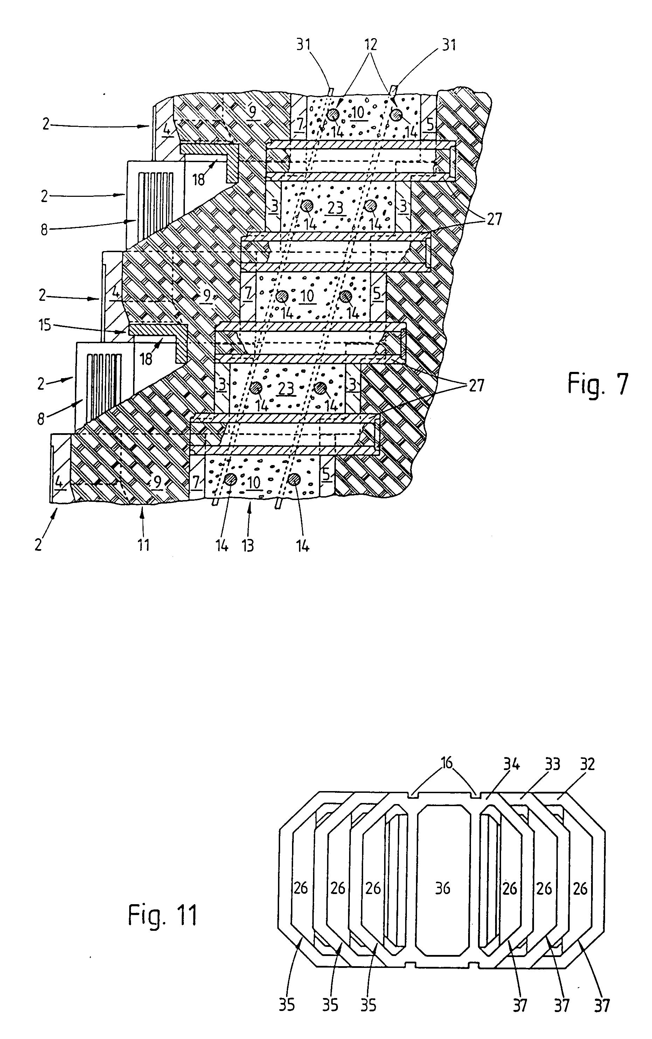

Als weitere Variante kann die freistehende Mauer aus Bauelementen, die sich von einer Maueraußenseite zur anderen erstrecken, aufgebaut werden. Fig. 11 zeigt drei übereinander angeordnete Bauelemente 32, 33, 34 einer solchen Mauer; zwischen den Bauelementen 32, 33, 34 liegen nicht sichtbare Verbindungsplattenpaare 3. Jedes der Bauelemente 32, 33, 34 hat drei hintereinander angeordnete Kammern 35, 36, 37. Die beiden Kammern 35 und 37 sind spiegelbildlich zueinander aufgebaut. Die beiden Kammern 35 und 36 bzw. 36 und 37 haben je eine gemeinsame Trennwand. Die Tiefenausdehnung der Bauelemente 32, 33, 34 ist unterschiedlich abgestuft und wird durch die Tiefe der Kammer 36 bestimmt. Für den Aufbau der freistehenden Mauer werden reihenweise Bauelemente gleicher Tiefenausdehnung, die durch die Verbindungsplatten 3 miteinander verbunden sind, verwendet. In der untersten Reihe liegen die Bauelemente 32 mit der größten Tiefenausdehnung. Die Tiefe der Bauelemente (nicht dargestellt) der nächst höheren Reihe ist um den doppelten Versatz der Reihe zur darunterliegenden Reihe kleiner. Die Bauelemente liegen immer auf je einer Seitenwand der beiden unter ihnen seitlich versetzten Bauelemente der darunterliegenden Reihe. Das Bauelement 33 in Fig. 11 liegt in der dritten and das Bauelement 34 in der fünften Reihe über dem Bauelement 32 der untersten Reihe. Nach dem Aufbau jeder Reihe können horizontale Armierungseisen 14, und nach der dritten, vierten oder fünften horizontalen Reihe vertikale Armierungseisen 31 durch die Kammern 36 und die Kammern zwischen den Bauelementen und den beiden Verbindungsplatten 3 geführt und anschließend mit Beton 13 ausgefüllt werden. Nach Abbinden des Beton 13 werden die Kammern 35 und 37 mit Erde 11 gefüllt und bepflanzt.

**Patentansprüche**

1. Verfahren zur Herstellung einer Mauer (1), insbesondere Stützmauer, aus hohlen Bauelementen (2), deren oben und unten offener Hohlraum durch mindestens eine von oben nach unten verlaufende Trennwand (7) in mindestens eine vordere und hintere Kammer (9, 10) unterteilt ist, bei dem die Bauelemente (2) versetzt aufeinander geschichtet und wenigstens teilweise mit Erde (11) gefüllt werden, wobei nur ein Teil der Kammern (9) an der Maueraussenseite liegt, dadurch gekennzeichnet, dass die Bauelemente (2) in einem Abstand nebeneinander angeordnet und durch mindestens eine unter Bildung eines Raums (23) formschlüssig mit ihnen zu verbindende vertikale Verbindungsplatte (3) miteinander verbunden werden und dass wenigstens ein Teil der nicht an der Maueraussenseite liegenden Kammern (10) und wenigstens ein Teil der Räume (23) zwischen den benachbarten Bauelementen (2) und der mindestens einen Verbindungsplatte (3) wenigstens teilweise mit Beton (13) gefüllt wird, wobei die formschlüssig angefügte mindestens eine Verbindungsplatte (3) ein Herausquellen des Betons (13) zwischen den versetzt darunterliegenden Bauelementen verhindert, und die an der Maueraussenseite liegenden Kammern (11) wenigstens teilweise mit Erde gefüllt sind.

2. Verfahren nach Anspruch 1, dadurch gekennzeichnet, dass die voneinander distanzierten Bauelemente (2) einer horizontalen Reihe über den Verbindungsplatten (3) der Bauelemente (2) der darunter liegenden horizontalen Reihe angeordnet werden.

3. Verfahren nach einem der Ansprüche 1 bis 2, dadurch gekennzeichnet, dass vor dem Betonieren Verbindungsmittel, vorzugsweise Armierungseisen (14), durch Oeffnungen (12) in der die mit Beton zu füllenden Kammern umschliessenden Wandung der Bauelemente (2) derselben horizontalen Reihe bzw. Reihen geführt und beim Betonieren eingebettet werden.

4. Verfahren nach den Ansprüchen 2 und 3, dadurch gekennzeichnet, dass vor dem Betonieren zweite Verbindungsmittel, vorzugsweise Armierungseisen (31), von oben nach unten durch die mit Beton (13) zu füllenden Kammern (10, 23) abwechselnd übereinander liegender Bauelemente (2) und Räume (23) zwischen benachbarten Bauelementen (2) und den Verbindungsplatten (3) geführt und vorzugsweise mit den ersten Verbindungsmitteln (14) zu einem Armierungsgitter verbunden werden.

5. Verfahren nach einem der Ansprüche 1 bis 4, dadurch gekennzeichnet, dass nach dem oder beim Verlegen jeder horizontalen Bauelementreihe Rohrstücke (27) wenigstens auf einen Teil der Bauelemente (2) in Aussparungen (20, 21) der der Maueraussenseite abgewandten Wand (5) und der die Kammern (9, 10) trennenden Trennwand (7) bzw. Trennwände, und/oder wenigstens auf einen Teil der aus zwei Verbindungsplatten (3) gebildeten Verbindungsplattenpaare (3) in Aussparungen (20) der Platten (3) gelegt werden, um hinter der Mauer (1) liegendes Erdreich mit der Erde (11) in der Kammer (9) an der Maueraussenseite zu verbinden.

6. Verfahren nach einem der Ansprüche 1 bis 6, dadurch gekennzeichnet, dass eine Bodenplatte (18) oder eine Bodenplatte (18) mit einem nach unten abgewinkelten Schenkel (28) in den unteren Teil der an der Maueraussenseite liegenden Kammer (9) derart eingelegt wird, dass die Bodenplatte (18) oder die Bodenplatte (18) mit dem nach unten abgewinkelten Schenkel (28) die Kammer (9) unten zur Maueraussenseite hin erddicht verschliesst und zur der Maueraussenseite abgewandten Seite der Kammer (9) mindestens eine Oeffnung frei lässt.

7. Verfahren nach einem der Ansprüche 1 bis 6, dadurch gekennzeichnet, dass die Bauelemente (2) bzw. die Bauelemente (2) und die Verbindungsplatten (3) zu einer freistehenden Mauer aufgeschichtet werden, wobei die Kammern an beiden Maueraussenseiten wenigstens teilweise mit Erde (11) gefüllt werden.

8. Bausatz zur Ausführung des Verfahrens nach Anspruch 1, umfassend hohle Bauelemente mit einem oben und unten offenen, durch eine Vorder- (4) und eine Rückwand (5) sowie zwei Seitenwände (6) begrenzten Hohlraum, der durch mindestens eine von oben nach unten verlaufende, senkrecht zu den Seitenwänden (6) stehende Trennwand (7) in mindestens eine vordere und eine hintere Kammer (9, 10) unterteilt ist, dadurch gekennzeichnet, dass zusätzlich Verbindungsplatten (3) vorgesehen sind und dass die Verbindungsplatten (3) und die Seitenwände (6) der Bauelemente (2) zur gegenseitigen formschlüssigen Verbindung an der der Trennwand (7) gegenüberliegenden Aussenseite der Seitenwände (6) derart ausgebildet sind, dass eine Mauer mit versetzt aufeinander geschichteten und mit Verbindungsplatten (3) verbundenen hohlen Bauelementen (2) aufgebaut werden kann, wobei zwischen den benachbarten und den versetzt darüber- bzw. darunterliegenden Bauelementen (2) und der dazwischen formschlüssig eingefügten Verbindungsplatte (3) ein abgedichteter, zumindest teilweise mit Beton füllbarer Raum (23) ensteht und wobei nur ein Teil der Kammern (9, 10) an der Maueraussenseite liegt und wenigstens teilweise mit Erde (17) füllbar ist und wenigstens ein Teil der nicht an der Maueraussenseite liegenden Kammern (10) wenigstens teilweise mit Beton füllbar ist.

9. Bausatz nach Anspruch 9, dadurch gekennzeichnet, dass der der Maueraussenseite gegenüberliegend anzuordnende Wandungsteil (5) des Bauelements an seiner Unterseite mindestens einen Vorsprung (17) als Montagehilfe hat.

10. Bausatz nach Anspruch 8 oder 9, dadurch gekennzeichnet, dass die an der Maueraussenseite anzuordnende Kammer (9) des Bauelements in ihrem unteren Teil mit einer Bodenplatte (18) oder einer Bodenplatte (18) mit einem nach unten abgewinkelten Schenkel (28) teilweise verschlossen oder verschliessbar ist.

11. Bausatz nach einem der Ansprüche 8 bis 10, dadurch gekennzeichnet, dass die Wandung des Bauelements (6) miteinander fluchtende, in die nicht an der Maueraussenseite anzuordnende Kammer (10) oder Kammern führende Oeffnungen (12, 20, 21) für Verbindungsmittel (14) aufweist, die dazu bestimmt sind, beim Einfüllen von Beton (13) in diese Kammer (10) oder Kammern eingebettet zu werden, um benachbarte Bauelemente (2) miteinander zu verbinden.

12. Bausatz nach einem der Ansprüche 8 bis 11, dadurch gekennzeichnet, dass am oberen und/oder unteren Rand der Trennwand (7) oder Trennwände und der gegenüberliegend der Maueraussenseite anzuordnenden Wandungsteile (5) in einer Linie je eine Aussparung (20, 21) zum Einlegen eines Rohrstückes (27) vorhanden ist.

13. Bausatz nach einem der Ansprüche 8 bis 12, dadurch gekennzeichnet, dass an der Verbindungsplatte (3) am oberen Rand ein nach hinten und am unteren Rand ein nach vorne gerichteter leistenförmiger Ansatz vorgesehen ist.

14. Bausatz nach einem der Ansprüche 8 bis 13, dadurch gekennzeichnet, dass an der Verbindungsplatte (3) am oberen und/oder unteren Rand eine Aussparung zum Einlegen eines Rohres vorgesehen ist.

15. Bausatz nach Anspruch 8, dadurch gekennzeichnet, dass die Verbindungsplatte (3) im wesentlichen dieselbe Länge wie die Trennwand (7) hat.

16. Bausatz nach Anspruch 8, dadurch gekennzeichnet, dass die Seitenwände (6) an ihren Aussenseiten gegenüber der Trennwand (7) je eine von oben nach unten verlaufende Nut (16) zum formschlüssigen Einfügen der Verbindungsplatten (3) aufweisen.

17. Mauer (1) hergestellt nach dem Verfahren gemäss Anspruch 1, mit versetzt aufeinander geschichteten hohlen Bauelementen (2), deren oben und unten offene Hohlräume durch mindestens je eine von oben nach unten verlaufende Trennwand (7) in mindestens eine vordere und hintere Kammer (9, 10) unterteilt sind, dadurch gekennzeichnet, dass die Bauelemente (2) in Abständen nebeneinander in annähernd horizontalen Reihen angeordnet sind, wobei benachbarte Bauelemente (2) in jeder der horizontalen Reihen unter Bildung eines Raums (23) durch mindestens eine vertikale Verbindungsplatte (3) verbunden sind, dass die an der Maueraussenseite (1) liegenden Kammern (9) wenigstens teilweise mit Erde (11) gefüllt und wenigstens ein Teil der anderen Kammern (10) wenigstens teilweise mit Beton (13) gefüllt und wenigstens ein Teil der Räume (23) zwischen den benachbarten Bauelementen (2) und der mindestens einen Verbindungsplatte (3) wenigstens teilweise mit Beton (13) ausgegossen sind.

18. Mauer nach Anspruch 17, hergestellt nach dem Verfahren gemäss Anspruch 4, bei der die Bauelemente (2) in Abständen nebeneinander in annähernd horizontalen Reihen angeordnet sind, dadurch gekennzeichnet, dass wenigstens ein Teil der betonierten Räume (23) zwischen den benachbarten Bauelementen (2) und den beiden Verbindungsplatten (3) horizontal und vertikal mit den betonierten Kammern (10) durch einbetonierte Verbindungsmittel (14, 31) verbunden ist.

19. Mauer nach einem der Ansprüche 17 oder 18, hergestellt nach dem Verfahren gemäss Anspruch 5 mit Bauelementen nach Anspruch 13, dadurch gekennzeichnet, dass mindestens in einem Teil der Aussparungen (20, 21) je ein Rohrstück (27) liegt, um hinter der Mauer (1) liegendes Erdreich mit der Erde (11) in den Kammern (9) an der Maueraussenseite zu verbinden.

20. Mauer nach einem der Ansprüche 17 bis 19, hergestellt nach dem Verfahren gemäss Anspruch 6, dadurch gekennzeichnet, dass die in die an der Maueraussenseite befindlichen Kammern (9) eingefüllte Erde (11) durch eine an der Unterseite dieser Kammern (9) eingelegte Bodenplatte (18) oder durch eine Bodenplatte (18) mit einem nach unten abgewinkelten Schenkel (28) am Durchsacken in die darunter liegenden Kammern (9) derart gehindert ist, dass eine Erdverbindung mit der unter oder der unter und über ihr mit Erde (11) gefüllten Kammer (9) an der Maueraussenseite besteht.

21. Mauer nach einem der Ansprüche 17 bis 20, dadurch gekennzeichnet, dass die Mauer frei steht und die an den beiden Maueraussenseiten liegenden Kammern (9) wenigstens teilweise mit Erde (11) gefüllt sind.

22. Mauer nach einem der Ansprüche 17 bis 21, dadurch gekennzeichnet, dass der mit Beton gefüllte Teil der Mauer sich mindestens über die halbe Mauerhöhe erstreckt.

**Claims**

1. Method for the production of a wall (1), in particular a supporting wall, of hollow construction elements (2) of which the hollow chamber, which is open at the top and bottom, is divided by means of at least one separating wall (7) extending from top to bottom into at least one front and one rear chamber (9, 10), in the case of which the construction elements (2) are stacked on one another in an offset manner and at least partially filled with earth (11), wherein only part of the chambers (9) is located at the external side of the wall, characterised in that the construction elements (2) are located adjacent one other at a distance and are connected by at least one vertical connection plate (3) which is to be connected thereto in a form-locking manner whilst forming a chamber (23); and in that at least part of the chambers (10) not at the external side of the wall, and at least part of the chambers (23) between the adjacent construction elements (2) and the connection plate (3), of which there is at least one, are at least partially filled with concrete (13), wherein the connection plate (3) of which there is at least one and which is attached in a form-locking manner prevents the concrete (13) flowing out between the construction components located therebelow in an offset manner, and the chambers (11) located at the external side of the wall are at least partially filled with earth.

2.  Method according to Claim 1, characterised in that the construction elements (2), which are distanced from one another, are arranged in a horizontal row above the connection plates (3) of the construction elements (2) of the horizontal row located therebelow.

3.  Method according to either of Claims 1 and 2, characterised in that, prior to the concreting process, connection means, preferably reinforcing irons (14), are guided through apertures (12) in the walls of the construction elements (2) in the same horizontal row or rows, which walls surround the chambers to be filled with concrete, and are embedded during the concreting process.

4.  Process according to Claims 2 and 3, characterised in that, prior to the concreting process, second connection means, preferably reinforcing irons (31), are guided from top to bottom through the chambers (10, 23), which are to be filled with concrete (13), of construction elements (2) located above one another in an alternating manner and chambers (23) between adjacent construction elements (2) and the connection plates (3), and are preferably connected to the first connection means (14) to form a reinforcing grid.

5.  Method according to any one of Claims 1 to 4, characterised in that after or during the positioning of each horizontal construction element row, pipe sections (27) are laid on at least one part of the construction elements (2) in recesses (20, 21) in the wall (5) facing away from the external side of the wall and in the separating wall (7) or separating walls separating the chambers (9, 10), and/or on at least part of the pair of connection plates (3) formed of two connection plates (3) in recesses (20) in the plates (3), in order to connect the ground behind the wall (1) to the earth (11) in the chamber (9) on the external side of the wall.

6.  Method according to any one of Claims 1 to 5, characterised in that a base plate (18) or a base plate (18) with a downwardly angled leg (28) is placed in the lower part of the chamber (9) located at the external side of the wall, in such a way that the base plate (18) or the base plate (18) with the downwardly angled leg (28) seals the chamber (9) at the bottom with respect to the external side of the wall and leaves at least one aperture open to the side of the chamber (9) facing away from the external side of the wall.

7.  Process according to any one of Claims 1 to 6, characterised in that the construction elements (2), or the construction elements (2) and the connection plates (3), are stacked to form a free-standing wall, wherein the chambers on both the external sides of the wall are at least partially filled with earth (11).

8.  Kit for performing the method according to Claim 1, comprising hollow construction elements with a hollow chamber that is open at the top and bottom, delimited by a front wall (4) and a rear wall (5) as well as two side walls (6), and is subdivided, by means of at least one separating wall (7) extending from the top to the bottom and standing perpendicular to the side walls (6), into at least one front and one rear chamber (9, 10) characterised in that, in addition, connection plates (3) are provided; and in that the connection plates (3) and the side wall (6) of the construction elements (2) are, for their mutual form-locking connection, formed in such a way on the external side of the side wall (6) opposite the separating wall (7), that a wall can be constructed with hollow construction elements (2) which are stacked on one another in an offset manner and are connected by connection plates (3), wherein a sealed chamber (23), which can be at least partially filled with concrete, is produced between the adjacent construction elements (2) and those that are located in an offset manner thereabove or therebelow and the connection plate (3) inserted in a form-locking manner therebetween, and wherein only part of the chambers (9, 10) is located at the external side of the wall and can be at least partially filled with earth (17) and at least part of the chamber (10) that is not on the external side of the wall may be at least partially filled with concrete.

9. Kit according to Claim 9, characterised in that the wall section (5) of the construction element to be disposed opposite the external side of the wall has on its lower side at least one projection (17) as a mounting aid.

10. Kit according to Claim 8 or Claim 9, characterised in that the chamber (9) of the construction element to be disposed at the external side of the wall, is partially closed or partially closeable in its lower part by means of a base plate (18) or by means of a base plate (18) with a downwardly angled leg (28).

11. Kit according to any one of Claims 8 to 10, characterised in that the wall of the construction element (6) has apertures (12, 20, 21) for connection means (14) which apertures are aligned with one another and lead into the chamber (10) or chambers that are not be located on the external side of the wall, the connection means (14) being intended to be embedded in this chamber (10) or these chambers when the concrete (13) is filled in, in order to connect adjacent construction elements (2) to one another.

12. Kit according to any one of Claims 8 to 11, characterised in that on the upper and/or lower edge of the separating wall (7) or separating walls and in the wall sections (5) that are to be located opposite the external side of the wall there is provided, in a row, one recess (20, 21) in each case for the pipe section (27) to be located.

13. Kit according to any one of Claims 8 to 12, characterised in that there is provided on the connection plate (3) at the upper edge, a strip-like attachment directed towards the rear and, at the lower edge, a strip-like attachment directed towards the front.

14. Kit according to any one of Claims 8 to 13, characterised in that there is provided on the upper and/or lower edge on the connection plate (3) a recess for the location of a pipe section.

15. Kit according to Claim 8, characterised in that the connection plate (3) is substantially the same length as the separating wall (7).

16. Kit according to Claim 8, characterised in that the side walls (6) each comprise, on the external side thereof that is opposite the separating wall (7), a groove (16) extending from the top to the bottom for the form-locking insertion of the connection plates (3).

17. Wall (1) produced according to the method according to Claim 1, with hollow construction elements (2) stacked on one another in an offset manner, of which the hollow chambers are open at the top and bottom and are subdivided into at least one front and one rear chamber (9, 10) by means of a separating wall (7) extending from the top to the bottom, characterised in that the construction elements (2) are arranged at a distance adjacent each other, in approximately horizontal rows, wherein neighbouring construction elements (2) in each of the horizontal rows are connected by at least one vertical connection plate (3), a chamber (23) being formed thereby; in that the chambers (9) on the external side of the wall (1) are at least partially filled with earth (11) and at least part of the other chambers (10) is at least partially filled with concrete (13) and at least part of the chambers (23) between the neighbouring construction elements (2) and the connection plate (3), of which there is at least one, are at least partially filled with concrete (13).

18. Wall according to Claim 17, produced by the method according to Claim 4, in the case of which the construction elements (2) are arranged at a distance adjacent one another, in approximately horizontal rows, characterised in that at least part of the concreted chambers (23) between the adjacent construction elements (2) and the two connection plates (3) is connected horizontally and vertically to the concreted chambers (10) by means of connection means (14, 31) that are concreted-in.

19. Wall according to Claim 17 or Claim 18, produced in accordance with the method according to Claim 5 with construction elements according to Claim 13, characterised in that, at least in part of the recesses (20, 21), there is located one pipe section (27) in each case, in order to connect the ground behind the wall (1) with the earth (11) in the chambers (9) on the external side of the wall.

20. Wall according to any one of Claims 17 to 19, produced in accordance with the process according to Claim 6, characterised in that the earth (11) that has been filled into the chambers (9) on the external side of the wall is prevented from sinking into the chambers (9) located therebelow by means of a base plate (18) inserted at the lower side of these chambers (9) or by means of a base plate (18) with a downwardly angled leg (28), in such a way that there is an earth connection with the chambers (9) that are filled with earth (11) and are located therebelow or thereabove on the external side of the wall.

21. Wall according to any one of Claims 17 to 20, characterised in that the wall is free-standing and in that the chambers (9) on both the external sides of the walls are at least partially filled with earth (11).

22. Wall according to any one of Claims 17 to 21, characterised in that the part of the wall that is filled with concrete extends over at least half the height of the wall.

## Revendications

1. Procédé de construction d'un mur (1), en particulier d'un mur d'appui, en éléments de construction creux (2), dont l'espace creux ouvert vers le haut et le bas est divisé par au moins une paroi de séparation (7), s'étendant de haut en bas, en au moins une chambre avant et une chambre arrière (9, 10), dans lequel les éléments de construction (2) sont empilés en étant décalés et sont remplis au moins partiellement de terre (11), seule une partie des chambres (9) reposant sur le côté extérieur du mur, caractérisé en ce que les éléments de construction (2) sont disposés à côté les uns des autres, à distance, et sont reliés entre eux par au moins une plaque de liaison verticale (3) à relier avec eux en engagement positif en formant un espace (23) et en ce qu'au moins une partie des chambres (10) qui ne sont pas situées sur le côté extérieur du mur et au moins une partie des espaces (23) entre les éléments de construction voisins (2) et au moins une plaque de liaison (3) sont remplis au moins partiellement de béton (13), la plaque de liaison (3) ajustée en engagement positif, empêchant le béton (13) de fuir entre les éléments de construction disposés au-dessous et décalés, les chambres (11) situées sur le côté extérieur du mur étant au moins partiellement remplies de terre.

2. Procédé selon la revendication 1, caractérisé en ce que les éléments de construction (2), distants entre eux, d'une rangée horizontale sont disposés au-dessus des plaques de liaison (3) des éléments de construction (2) de la rangée horizontale disposée au-dessous.

3. Procédé selon l'une des revendications 1 et 2, caractérisé en ce que des moyens de liaison, de préférence des fers d'armature (14), sont introduits par des ouvertures (12) avant le bétonnage dans la paroi des éléments de construction (2) d'une rangée ou de rangées horizontales, qui entourent les chambres à remplir de béton, et sont encastrés lors du bétonnage.

4. Procédé selon les revendications 2 et 3, caractérisé en ce que des deuxièmes moyens de liaison, de préférence des fers d'armature (31), sont introduits de haut en bas avant le bétonnage à travers les chambres (10, 23) à remplir de béton (13) d'éléments de construction (2) superposés en alternance et à travers les espaces (23) à remplir de béton (13) entre des éléments de construction voisins (2) et les plaques de liaison (3),et sont de préférence reliés aux premiers moyens de liaison (14) pour former un treillis d'armature.

5. Procédé selon l'une des revendications 1 à 4, caractérisé en ce que l'on dispose, avant ou pendant la pose de chaque rangée horizontale d'éléments de construction, afin de relier le terrain situé derrière le mur (1) à la terre (11) des chambres (9) situées sur le côté extérieur du mur, des éléments tubulaires (27), au moins sur une partie des éléments de construction (2) dans des évidements (20, 21) ménagés dans la paroi (5) opposée au côté extérieur du mur et dans la paroi de séparation ou les parois de séparation (7), séparant les chambres (9, 10) et/ou au moins sur une partie de paires (3) de plaques de liaison constituées par deux plaques de liaison (3), dans des évidements (20) ménagés dans ces plaques (3).

6. Procédé selon l'une des revendications 1 à 6, caractérisé en ce qu'une plaque de fond (18), ou une plaque de fond (18) pourvue d'une branche (28) coudée vers le bas, est introduite dans la partie inférieure de la chambre (9) située sur le côté extérieur du mur d'une manière telle que la plaque de fond (18), ou la plaque de fond (18) pourvue de la branche (28) coudée vers le bas, ferme la chambre (9) d'une manière étanche avec la terre, au-dessous du côté extérieur de mur, et laisse libre au moins une ouverture vers le côté de la chambre (9), opposé au côté extérieur du mur.

7. Procédé selon l'une des revendications 1 à 6, caractérisé en ce que les éléments de construction (2), ou les éléments de construction (2) et les plaques de liaison (3), sont empilés en un mur isolé, les chambres des deux côtés extérieurs de mur étant au moins partiellement remplies de terre (11).

8. Ensemble d'éléments destiné à la mise en oeuvre du procédé selon la revendication 1, comprenant des éléments de construction creux où est ménagé un espace creux ouvert vers le haut et vers le bas, limité par une paroi avant (4) et une paroi arrière (5) ainsi que par deux parois latérales (6), qui est divisé par au moins une paroi de séparation (7) s'étendant de haut en bas, disposée perpendiculairement aux parois latérales (6), en au moins une chambre avant et une chambre arrière (9, 10), caractérisé en ce qu'il est en outre prévu des plaques de liaison (3) et en ce que les plaques de liaison (3) et les parois latérales (6) des éléments de construction (2) sont formées de manière à constituer, sur le côté extérieur des parois latérales (6) opposé à la paroi de séparation (7), une liaison mutuelle en engagement positif telle que l'on peut construire un mur à l'aide d'éléments de construction creux (2) empilés de façon décalée et reliés à des plaques de liaison (3), un espace (23) rendu étanche, qui peut être rempli au moins partiellement de béton, étant réalisé entre les éléments de construction (2) voisins et ceux qui sont disposés au-dessous et au-dessus d'eux en étant décalés et la plaque de liaison (3) insérée entre eux en engagement positif, seule une partie des chambres (9, 10) étant située sur le côté extérieur du mur et pouvant être au moins partiellement remplie de terre (17), au moins une partie des chambres (10) qui n'est pas située sur le côté extérieur du mur pouvant être au moins partiellement remplie de béton.

9. Ensemble d'éléments selon la revendication 9, caractérisé en ce que la partie de paroi (5) de l'élément de construction qui doit être situé de façon opposée au côté extérieur du mur comprend au moins une saillie (17) sur son côté inférieur, pour faciliter son montage.

10. Ensemble d'éléments selon la revendication 8 ou 9, caractérisé en ce que la chambre 9 de l'élément de construction qui doit être disposée sur le côté extérieur du mur est, ou peut être, partiellement fermée à sa partie inférieure par une plaque de fond (18), ou par une plaque de fond (18) pourvue d'une branche (28) coudée vers le bas.

11. Ensemble d'éléments selon l'une des revendications 8 à 10, caractérisé en ce que la paroi de l'élément de construction (6) comprend des ouvertures (12, 20, 21), alignées entre elles, conduisant dans la chambre ou les chambres (10) qui ne sont pas à disposer sur le côté extérieur du mur, et destinées à des moyens de liaison (14) prévus pour être encastrés dans cette chambre ou ces chambres (10), lorsque l'on coule le béton (13), afin de relier entre eux des éléments de construction voisins.

12. Ensemble d'éléments selon l'une des revendications 8 à 11, caractérisé en ce qu'il est ménagé selon une ligne, pour l'introduction d'un élément tubulaire (27), une série d'évidements (20, 21) formés chacun dans une des parties de paroi (5) sur le bord supérieur et/ou inférieur de la paroi ou des parois de séparation (7) et des parties (5) de paroi à disposer sur le côté opposé au côté extérieur du mur.

13. Ensemble d'éléments selon l'une des revendications 8 à 12, caractérisé en ce qu'il est prévu, sur la plaque de liaison (3), un appendice en forme de baguette, tourné vers l'arrière sur le bord supérieur, et un appendice similaire tourné vers l'avant sur le bord inférieur.

14. Ensemble d'éléments selon l'une des revendications 8 à 13, caractérisé en ce qu'il est prévu sur la plaque de liaison (3), sur le bord supérieur et/ou inférieur, un évidement pour l'insertion d'un tube.

15. Ensemble d'éléments selon la revendication 8, caractérisé en ce que la longueur de la plaque de liaison (3) est sensiblement égale à celle de la paroi de séparation (7).

16. Ensemble d'éléments selon la revendication 8, caractérisé en ce que les parois latérales (6) présentent chacune, sur leurs côtés extérieurs, face à la paroi de séparation (7), une encoche (16) s'étendant de haut en bas en vue d'une insertion des plaques de liaison (3) en engagement positif.

17. Mur (1) fabriqué selon le procédé conforme à la revendication 1, comportant des éléments de construction creux (2) empilés en étant décalés, dont les espaces creux ouverts vers le haut et vers le bas sont divisés chacun par au moins une paroi de séparation s'étendant de haut en bas en au moins une chambre avant et une chambre arrière (9, 10), caractérisé en ce que les éléments de construction (2) sont disposés à côté l'un de l'autre, distants entre eux, dans des rangées à peu près horizontales, des éléments de construction voisins (2) de chacune des rangées horizontales étant reliés par au moins une plaque de liaison verticale (3) en formant un espace (23), en ce que les chambres (9) situées sur le côté extérieur (1) du mur sont au moins partiellement remplies de terre (11) et qu'au moins une partie des autres chambres (10) est au moins partiellement remplie de béton (13), et en ce qu'au moins une partie des espaces (23) entre les éléments de construction voisins (2) et la plaque de liaison (3) est au moins partiellement rempli de béton coulé (13).

18. Mur selon la revendication 17, fabriqué selon le procédé conforme à la revendication 4, dans lequel les éléments de construction (2) sont disposés à côté l'un de l'autre, distants entre eux, dans des rangées à peu près horizontales, caractérisé en ce qu'au moins une partie des espaces bétonnés (23) entre les éléments de construction voisins (2) et les deux plaques de liaison (3) sont reliés horizontalement et verticalement aux chambres bétonnées (10) par des moyens de liaison (14, 31), encastrés dans le béton.

19. Mur selon l'une des revendications 17 ou 18, fabriqué selon le procédé conforme à la revendication 5 à l'aide d'éléments de construction conformes à la revendication 13, caractérisé en ce qu'il est respectivement disposé, dans chacun d'au moins une partie des évidements (21), un élément tubulaire (27) pour relier le terrain situé derrière le mur (1) à la terre (11) des chambres (9), situées sur le côté extérieur du mur.

20. Mur selon l'une des revendications 17 à 19, fabriqué selon le procédé conforme à la revendication 6, caractérisé en ce que la terre (11) qui remplit les chambres (9) situées sur le côté extérieur du mur est empêchée, par une plaque de fond (18), ou une plaque de fond (18) pourvue de branches (28) coudées vers le bas, de tomber à plat dans les chambres (9) situées au-dessous, de sorte qu'il est réalisé sur le côté extérieur du mur une liaison de la terre avec celle de la chambre (9) remplie de terre située au-dessous d'elle ou avec celle, des chambres remplies de terre situées tant au-dessous qu'au-dessus d'elle,.

21. Mur selon l'une des revendications 17 à 20, caractérisé en ce que le mur est isolé et en ce que les chambres (9) situées sur les deux côtés extérieurs de mur sont au moins partiellement remplies de terre (11).

22. Mur selon l'une des revendications 17 à 21, caractérisé en ce que la partie du mur remplie de béton s'étend au moins sur la moitié de la hauteur du mur.

1/3

Fig. 1

Fig. 2

Fig. 3

EP 0 322 667 B1

Fig. 4

Fig. 5

Fig. 6

Fig. 8

Fig. 9

Fig. 10

Fig. 7

Fig. 11